# EUROPÄISCHE PATENTSCHRIFT

(11) **EP 2 959 868 B1**
(45) Veröffentlichungstag und Bekanntmachung des Hinweises auf die Patenterteilung: **31.05.2017**
(21) Anmeldenummer: 14173966.4
(22) Anmeldetag: 25.06.2014
(51) Int. Cl.: A61F 5/01

(54) **Knieorthese zur Stützung eines Kniegelenks**
Knee brace for supporting a knee joint
Orthèse de genou destinée au soutien d'une articulation du genou

(43) Veröffentlichungstag der Anmeldung: 30.12.2015
(73) Patentinhaber: Ortho-Team AG, 3008 Bern (CH)
(72) Erfinder: Bosshard, Adrian, 3065 Bolligen (CH); Kohl, Sandro, 3007 Bern (CH)
(74) Vertreter: BOVARD AG

(56) Entgegenhaltungen:
- DE-A1- 10 259 751
- DE-A1- 19 811 925
- US-A- 5 002 045
- US-A1- 2011 098 618
- US-A1- 2013 110 020

## Beschreibung

Die vorliegende Erfindung bezieht sich auf eine Knieorthese zur Stützung eines Kniegelenks bei einer Instabilität, umfassend einen am Oberschenkel über Befestigungsmittel befestigbaren Oberschenkelteil mit einer oberen Halbschale und an der Halbschale befestigte Gelenkplatten, einen am Unterschenkel über Befestigungsmittel befestigbaren Unterschenkelteil mit einer unteren Halbschale und seitlich an der unteren Halbschale angebrachten Schienenelemente, welche Schienenelemente über Gelenkmittel mit der jeweiligen Gelenkplatte verbunden sind und in welchen Schienenelementen an den den Gelenkmitteln abgewandten Bereichen die untere Halbschale um eine Schwenkachse schwenkbar gelagert ist und über Stellmittel gegen den Unterschenkel hin schwenkbar ist. Die Patentveröffentlichung US 2013/110020 A1 offenbart eine Knieorthese mit der Stellmittel die aus mindestens einem längsstabilen flexiblen Zugelement gebildet sind.

Insbesondere bei Knieinstabilitäten aufgrund einer Verletzung der Kreuzbänder ist es erforderlich, dass das Kniegelenk gestützt wird, um insbesondere eine Fehlstellung der Tibia zum Femur zu vermeiden. Hierzu werden Knieorthesen eingesetzt, die dem Kniegelenk die gewünschte Stabilität geben. Es hat sich nun gezeigt, dass mit derartigen Knieorthesen insbesondere beim gebogenen Knie, wie es beispielsweise in einer Sitzposition der entsprechenden Person auftritt, sich trotzdem eine Verschiebung der Tibia zum Femur ergibt. Eine derartige Verschiebung kann eine Überdehnung der entsprechenden Kreuzbänder zur Folge haben, was sowohl bei einer operativen als auch bei einer konservativen Behandlung der Verletzung auftreten kann und möglichst vermieden werden soll.

Aus der Patentveröffentlichung DE 102 59 751 A1 ist eine Knieorthese entnehmbar, welche eine derartige Verschiebung der Tibia zum Femur unterdrücken soll. Hierzu ist diese Knieorthese so ausgebildet, dass die untere Halbschale der Knieorthese über einen einstellbaren Federdruck gegen den oberen Bereich des Unterschenkels gedrückt wird, um dadurch einem Bewegen der Tibia zum Femur entgegen zu wirken.

Hierbei hat es sich gezeigt, dass dieser Druck gegen den oberen Bereich des Unterschenkels nicht optimal ist, so kann beispielsweise dieser Druck in der gebeugten Position des Knies die erwünschte Grösse aufweisen, in der gestreckten Position des Knies wird dieser Druck aber zu gross, was nachteilig ist.

Die Aufgabe der vorliegenden Erfindung besteht nun darin, dass eine Knieorthese geschaffen wird, welche die oben genannten Nachteile nicht aufweist, und bei welcher der Druck in ventraler oder dorsaler Richtung auf den oberen Bereich des Unterschenkels in Abhängigkeit der Biegung des Knies angepasst wird.

Erfindungsgemäss erfolgt die Lösung dieser Aufgabe durch die Merkmale des Anspruchs 1. Vorteilhafte Weiterbildungen sind in den abhängigen Ansprüchen beschrieben. Anspruch 1 beschreibt auch, dass die Stellmittel aus mindestens einem längsstabilen flexiblen Zugelement gebildet sind, dessen eine Endbereich an einem bezüglich der Schienenelemente schwenkbaren Teil der Gelenkmittel mittels einer ersten Halterung gehalten ist, dessen anderer Bereich an der unteren Halbschale mittels einer zweiten Halterung gehalten ist und welches längsstabile flexible Längselement zwischen der ersten Halterung und der zweiten Halterung um ein am Schienenelement angebrachtes Umlenkelement umgelenkt ist.

Mit dieser Ausgestaltung der Knieorthese kann erreicht werden, dass der Druck auf den oberen Bereich des Unterschenkels beim Biegen des Knies kontinuierlich ansteigt, und dadurch dem Knie in gestreckter und in jeder gebogenen Position die optimale Stabilität verleiht. Mit dieser erfindungsgemässen Knieorthese wird das Kniegelenk derart gestützt, dass das entsprechende Kreuzband in der anatomisch richtigen Position gehalten wird, wodurch auch ein Anwachsen der Sehnenstümpfe erreicht werden kann.

In vorteilhafter Weise sind die Gelenkmittel jeweils als Viergelenksystem ausgebildet, und umfassen einen ersten Schenkel und einen zweiten Schenkel, welche jeweils an der Gelenkplatte und am entsprechenden Schienenelement angelenkt sind. Mit diesem Viergelenksystem kann die Gleit-Rollbewegung des Kniegelenks nachgebildet werden, wodurch die Knieorthese das Kniegelenk in optimaler Weise unterstützt.

Die erste Halterung zum Halten des längsstabilen flexiblen Zugelementes ist am ersten Schenkel des Viergelenksystems angebracht, wodurch der Schwenkweg, den die untere Halbschale zur Ausübung des Drucks auf den oberen Bereich des Unterschenkels ausüben soll, optimal erreichbar ist.

In vorteilhafter Weise ist die erste Halterung zum Halten des längsstabilen flexiblen Zugelements verstellbar am ersten Schenkel des Viergelenksystems angebracht, wodurch der Schwenkweg der unteren Halbschale einstellbar und in optimaler Weise an das zu stützende Kniegelenk anpassbar ist.

In vorteilhafter Weise ist zur Verstellung der ersten Halterung am ersten Schenkel eine schlitzförmige Ausnehmung angebracht, entlang welchem die erste Halterung verschiebbar und in der eingestellten Position fixierbar ist, was eine einfache Herstellung und eine einfache Bedienung ermöglicht.

In vorteilhafter Weise ist die erste Halterung in einem Gleitschuh angebracht, welcher entlang der schlitzförmigen Ausnehmung verschiebbar ist und durch Klemmmittel fixierbar ist. Durch diese Ausgestaltung ergibt sich eine konstruktiv einfache Lösung der Verstellmöglichkeit und eine gute Bedienbarkeit.

In vorteilhafter Weise ist entlang der schlitzförmigen Ausnehmung eine Skala angeordnet, wodurch die Einstellung in einfacher Weise und reproduzierbar vorgenommen werden kann.

Eine weitere vorteilhafte Ausgestaltung der Erfindung besteht darin, dass in das längsstabile flexible Längselement mindestens ein Federelement eingesetzt ist. Durch dieses Federelement können Druckspitzen, die auf den oberen Bereich des Unterschenkels wirken könnten, vermieden werden, was den Tragkonfort für diese Knieorthese verbessert.

Eine weitere vorteilhafte Ausgestaltung der Erfindung besteht darin, dass die Länge des längsstabilen flexiblen Elements über eine Längeneinstellungseinrichtung einstellbar ist. Dadurch lässt sich diese Knieorthese in optimaler Weise an den jeweiligen Patienten anpassen, beispielsweise an unterschiedliche Ausbildungen der Muskulatur.

In vorteilhafter Weise ist die Längeneistellungseinrichtung in die zweite Halterung integriert und als drehbarer Knopf ausgebildet, dessen Achse in der unteren Halbschale gelagert ist und um welche das längsstabile flexible Element wickelbar ist. Dadurch wird eine einfache konstruktive Ausgestaltung dieser Längeneistellungseinrichtung erhalten, die auch einfach zu bedienen ist.

In vorteilhafter Weise sind die Befestigungsmittel für die obere und untere Halbschale als Bänder mit Klettverschluss ausgebildet, wodurch diese Knieorthese in optimal angepasster Weise am Bein der Person befestigt werden kann, bei welcher eine Stützung des Kniegelenks erforderlich ist.

Eine weitere vorteilhafte Ausgestaltung der Erfindung besteht darin, dass das längsstabile flexible Element ein Kabel ist, vorzugsweise aus faserverstärkten Kunststoff. Dadurch können gute Gleiteigenschaften erhalten werden, die vorteilhaft sind, wenn das Umlenkelement mit einer Gleitfläche ausgestattet ist, zusätzlich wird einer Verschmutzung vorgebeugt.

Eine Ausführungsform der Erfindung wird nachfolgend an Hand der beiliegenden Zeichnung beispielhaft näher erläutert.

Es zeigt
Figur 1 in schematischer Darstellung eine erfindungsgemässe Knieorthese im an einem Bein befestigten Zustand, bei gestrecktem Knie;
Figur 2 die Knieorthese gemäss Figur 1 bei leicht gebeugtem Knie;
Figur 3 die Knieorthese gemäss Figur 1 bei stark gebeugtem Knie;
Figur 4 in räumlicher Darstellung das Viergelenksystem der Knieorthese mit längsstabilem flexiblem Zugelement;
Figur 5 in räumlicher Darstellung das Viergelenksystem gemäss Figur 4, wobei die nicht sichtbaren Linien gestrichelt dargestellt sind;
Figur 6 in räumlicher Darstellung die Verstelleinrichtung, mittels welcher die erste Halterung im ersten Schenkel des Viergelenksystems verstellt werden kann;
Figur 7 eine Ansicht auf die Verstelleinrichtung für die erste Halterung gemäss Figur 6;
Figur 8 eine Schnittdarstellung durch die Verstelleinrichtung für die erste Halterung entlang Linie VIII-VIII gemäss Figur 7; und
Figur 9 in räumlicher Darstellung die Ansicht eines Gleitschuhs für die Verstelleinrichtung.

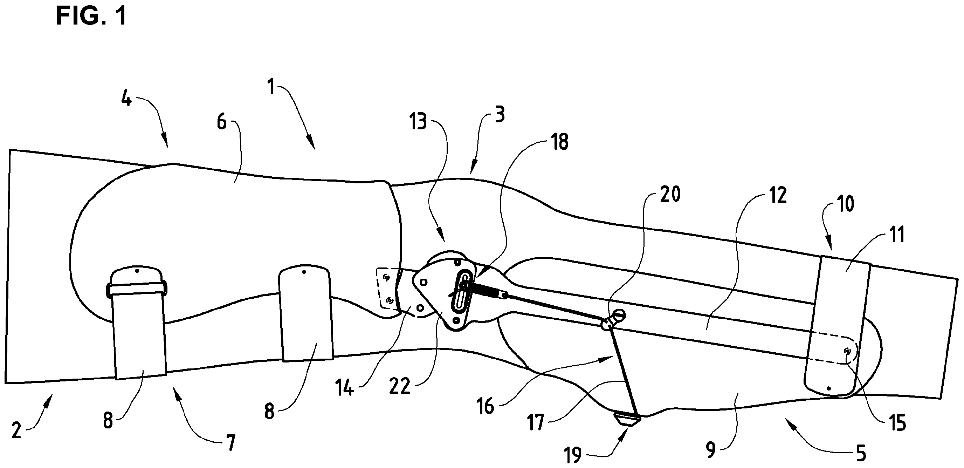

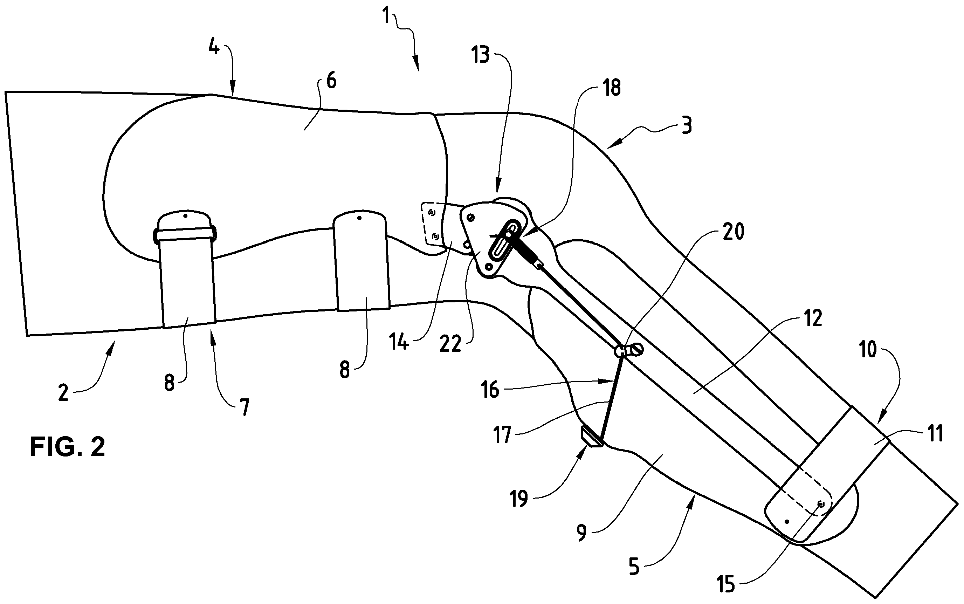

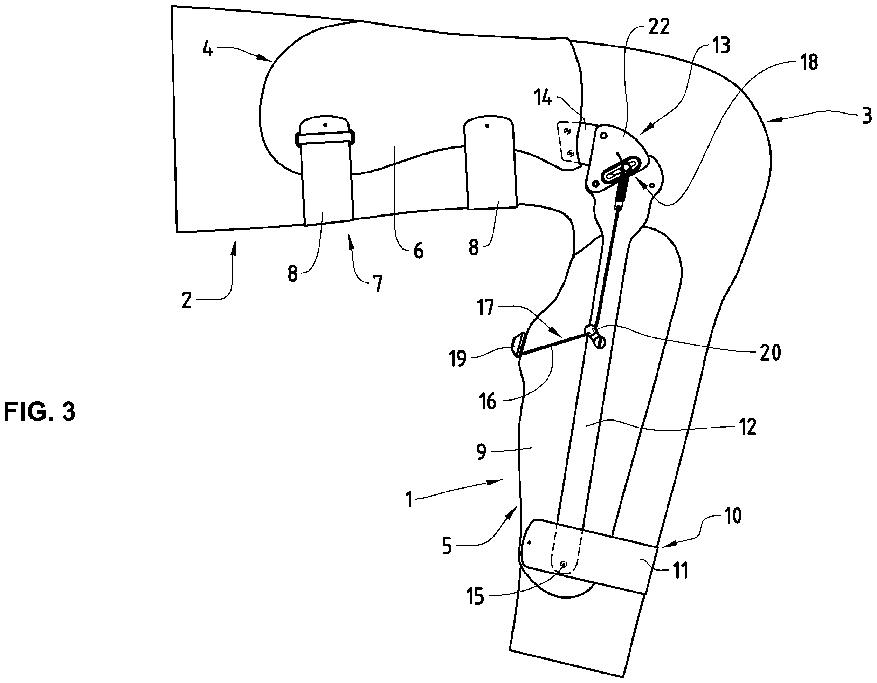

Aus den Figuren 1 bis Figur 3 ist eine erfindungsgemässe Knieorthese 1 ersichtlich die am entsprechenden Bein 2 des zu stützenden Knies 3 befestigt ist. Hierbei zeigt Figur 1 das Bein 2 mit gestreckten Knie 3, Figur 2 das Bein 2 mit leicht gebeugten Knie 3 und Figur 3 das Bein 2 mit stark gebeugten Knie 3. Die Knieorthese 1 besteht hierbei aus einem Oberschenkelteil 4 und einem Unterschenkelteil 5. Der Oberschenkelteil 4 besteht in bekannter Weise aus einer oberen Halbschale 6, welche in bekannter Weise von vorne auf den Oberschenkel aufgesetzt und über Befestigungsmittel 7 am Oberschenkel befestigt werden kann. Diese Befestigungsmittel 7 können in bekannter Weise die Form von Bändern 8 aufweisen, die in bekannter, nicht dargestellter Weise mit Klettverschlüssen versehen sein können, wodurch eine einfach und optimal Befestigung ermöglicht wird.

Der Unterschenkelteil 5 wird durch eine untere Halbschale 9 gebildet. Diese ist von hinten um den Unterschenkel gelegt. Über ein weiteres Befestigungsmittel 10, das in bekannter Weise wiederum aus einem Band 11 mit Klettverschluss gebildet ist, wird der untere Bereich der unteren Halbschale 9 am Unterschenkel fixiert.

Auf beiden Seiten der unteren Halbschale 9 ist jeweils ein Schienenelement 12 angebracht, deren eines Ende über Gelenkmittel 13 mit einer Gelenkplatte 14 gelenkig verbunden sind, welche Gelenkplatte 14 an der oberen Halbschale befestigt ist. Über diese Gelenkmittel 13, welche später noch im Detail beschrieben werden, sind die Schienenelemente 12 gemeinsam mit der unteren Halbschale 9 und die obere Halbschale 6 im Bereich des Kniegelenks, das durch die Gelenkmittel 13 nachgebildet wird, gelenkig verbunden. Die den Gelenkmitteln 13 abgewandten Bereiche der Schienenelemente 12 sind an der unteren Halbschale 6 schwenkbar gelagert und um eine Schwenkachse 15 schwenkbar.

Über Stellmittel 16 kann nun die untere Halbschale 9 um die Schwenkachse 15 in ventraler Richtung gegen den oberen Teil des Unterschenkels gedrückt werden. Die Stellmittel 16 bestehen hierbei aus einem längsstabilen flexiblen Zugelement 17, dessen eine Endbereich mittels einer ersten Halterung 18 an einem schwenkbaren Teil der Gelenkmittel 13 gehalten ist, wie später noch gesehen wird. Der andere Bereich dieses längsstabilen flexiblen Zugelementes 17 ist mittels einer zweiten Halterung 19 an der unteren Halbschale 9 gehalten. Etwa auf der Höhe der zweiten Halterung 19 ist am jeweiligen Schienenelement 12 ein Umlenkelement 20 angebracht, um welches das längsstabile flexible Zugelement 17 zwischen der ersten Halterung 18 und der zweiten Halterung 19 gleitend umgelenkt wird. Das längsstabile flexible Zugelement 17 kann hierbei als Kabel ausgebildet sein, bestehend aus Kunststoff mit einer glatten Oberfläche, erforderlichenfalls noch verstärkt mit Fasern, so dass die Reibung im Umlenkelement 20 möglichst gering gehalten werden kann. Selbstverständlich sind auch andere bekannte und geeignete längsstabile flexible Zugelemente 17 denkbar und einsetzbar.

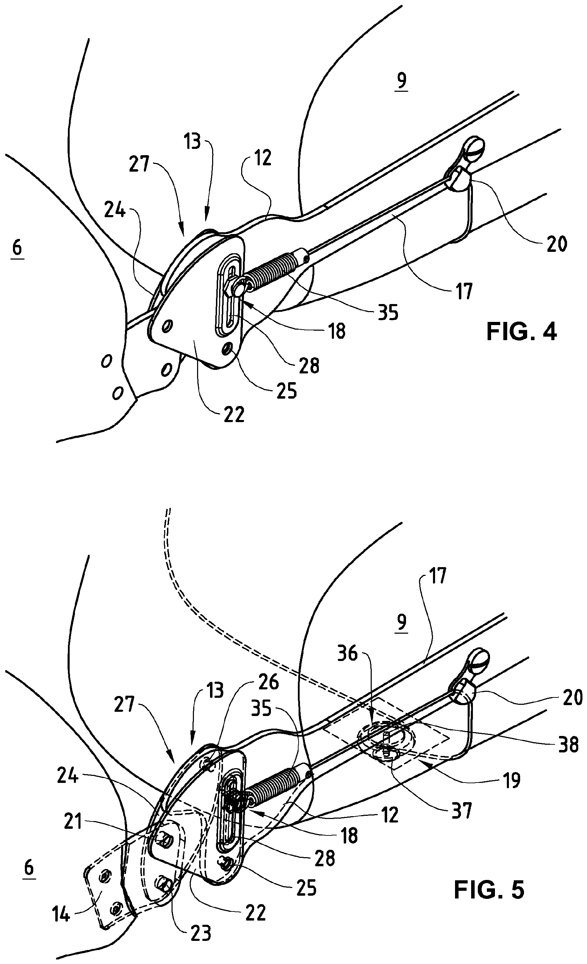

In den Figuren 4 und 5 sind die Gelenkmittel 13 dargestellt, die als Viergelenksystem 27 ausgebildet sind. Dieses Viergelenksystem 27 umfasst die Gelenkplatte 14, die an der oberen Halbschale 6 befestigt ist. Über ein erstes Schwenklager 21 ist ein erster Schenkel 22 mit der Gelenkplatte 14 schwenkbar verbunden. Über ein zweites Schwenklager 23, das ebenfalls an der Gelenkplatte 14 angeordnet ist, ist ein zweiter Schenkel 24 mit der Gelenkplatte 14 schwenkbar verbunden. Der erste Schenkel 22 ist über ein drittes Schwenklager 25 schwenkbar mit dem oberen Teil des Schienenelements 12 verbunden, während über ein viertes Schwenklager 26 der zweite Schenkel 24 ebenfalls mit dem oberen Bereich des Schienenelements 12 schwenkbar verbunden ist. Wie aus den Figuren 4 und 5 ersichtlich ist, kreuzen sich der erste Schenkel 22 und der zweite Schenkel 24. Durch diese bekannte Viergelenksystemanordnung wird die Gleit-Rollbewegung des Kniegelenks in optimaler Weise nachgebildet.

Wie ebenfalls aus Figur 4 und Figur 5 ersichtlich ist, ist die erste Halterung 18 für das längsstabile flexible Zugelement 17 am ersten Schenkel 22 dieses Viergelenksystems 27 angeordnet. Beim Biegen der Knieorthese wird der erste Schenkel 22 um das dritte Schwenklager 25 bezüglich des Schienenelements 12 leicht im Uhrzeigersinn verschwenkt, wodurch ein Zug auf das längsstabile flexible Zugelement 17 ausgeübt wird, wie das nachfolgend beschrieben wird.

In Figur 1 befindet sich das Knie und somit die Knieorthese in gestreckter Lage, über das längsstabile flexible Element 17 nimmt die untere Halbschale bezüglich der Schienenelemente 12 eine in Figur 1 dargestellte Position ein. Bei einer leichten Biegung des Kniegelenks und somit der Knieorthese 1 wird das längsstabile flexible Zugelement 17 durch die Anordnung der ersten Halterung 18, wie vorgängig beschrieben wurde, Richtung Oberschenkel gezogen. Über das Umlenkelement 20 zieht das längsstabile flexible Zugelement 17 die untere Halbschale 9, schwenkbar um die Schwenkachse 15, in ventraler Richtung gegen den Unterschenkel hin, der Unterschenkel bzw. die Tibia wird somit bezüglich dem Femur ebenfalls in ventraler Richtung gedrückt, was zu einer Entlastung des hinteren Kreuzbandes führt.

Bei einer starken Biegung des Kniegelenks und der Knieorthese wird durch das weitere Verschwenken des ersten Schenkels 22 bezüglich der Schienenelemente 12 die untere Halbschale 9 um die Schwenkachse 15 noch stärker gegen den Unterschenkel gedrückt, was bei einem stark gebeugten Knie eine optimale Entlastung auf das hintere Kreuzband ergibt. Beim Strecken des Knies nimmt der Druck der unteren Halbschale 9 auf den Unterschenkel wieder ab, man erreicht wieder die Ausgangsposition.

Wie insbesondere aus den Figuren 4 und 5 ersichtlich ist, ist die jeweilige erste Halterung 18 verstellbar am ersten Schenkel 22 des Viergelenksystems 27 angeordnet. Hierzu ist am ersten Schenkel 22 eine schlitzförmige Ausnehmung 28 angebracht, entlang welcher die erste Halterung 18 verschiebbar und in der eingestellten Position fixierbar ist, wie das nachfolgend beschrieben wird.

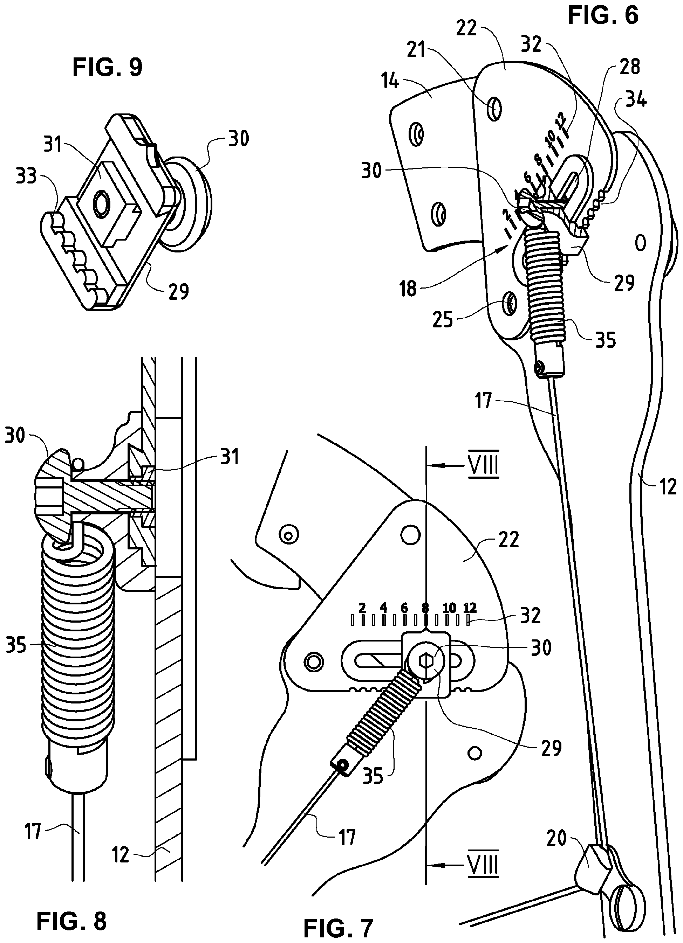

Wie aus den Figuren 6 bis 9 ersichtlich ist, besteht diese erste Halterung 18 aus einem Gleitschuh 29, welcher entlang der schlitzförmigen Ausnehmung 28 verschiebbar ist. In bekannter Weise ist an diesem Gleitschuh 29 eine Klemmschraube 30 angebracht, die in ein Spannelement 31 eingeschraubt ist. Über die Klemmschraube 30 und das Spannelement 31 kann der Gleitschuh 29 in der eingestellten Position in der schlitzförmigen Ausnehmung 28 fixiert werden. An ersten Schenkel 22 ist entlang der schlitzförmigen Ausnehmung 28 eine Skala 32 angeordnet, über diese Skala 32 kann der Gleitschuh 29 in der gewünschten Position und reproduzierbar eingestellt werden. Zusätzlich können an diesem Gleitschuh 29 noch Zähne 33 angebracht sein, die in eine entsprechende Verzahnung 34, die am ersten Schenkel 22 längs der schlitzförmigen Ausnehmung 28 angebracht ist, einklinken können, was die Einstellung erleichtet und die Fixierung verbessert. Am Gleitschuh 29 kann das längsstabile flexible Zugelement 17 eingehängt werden. Durch diese Verstellbarkeit kann der Schwenkweg der ersten Halterung 18 um das dritte Schwenklager 25 vergrössert oder verkleinert werden, wodurch entsprechend der Verschwenkweg der unteren Halbschale 9 vergrössert oder verkleinert wird. Dadurch kann der Druck, den die untere Halbschale 9 auf den Unterschenkel in ventraler Richtung ausüben soll, entsprechend vergrössert oder verkleinert werden, eine Anpassung an die Körperkonstellation ist somit problemlos möglich, je nachdem wie beispielsweise die Muskulatur des Trägers ausgebildet ist.

Wie insbesondere aus den Figuren 4 bis 8 ersichtlich ist, ist am Endbereich des längsstabilen flexiblen Zugelementes, das in den ersten Halterung 18 gehalten wird, ein Federelement 35 eingesetzt, das im vorliegenden Ausführungsbeispiel als Spiralfeder ausgebildet ist. Mit diesem Federelement 35 erhält man eine gewisse Elastizität des längsstabilen flexiblen Zugelementes 17, dadurch können übermässige Drücke auf den Unterschenkel abgefedert werden. Durch Anbringen einer Markierung kann ein maximaler Federweg festgelegt werden, bei dessen Überschreitung die Zugkraft des Federelementes zu gross würde. Die Federelemente sind austauschbar, es können jeweils Federelemente mit den gewünschten Anforderungen entsprechenden Federkennlinien eingesetzt werden.

Wie insbesondere aus Figur 5 ersichtlich ist, ist die zweite Halterung 19 für das längsstabile flexible Zugelement 17 an der unteren Halbschale 9 mit einer Längeneinstellungseinrichtung 36 versehen. Diese Längeneinstellungseinrichtung 36 kann aus einem drehbaren Knopf 37 gebildet sein, dessen Achse 38 in der unteren Halbschale 9 gelagert ist, und um welche die beiden Enden des längsstabilen flexiblen Zugelementes 17 wickelbar sind. Über diese Längeneinstellungseinrichtung 36 kann die Grundposition der unteren Halbschale 9 eingestellt und an die körperliche Konstellation des Trägers dieser Knieorthese angepasst werden, beispielsweise je nachdem, wie die Muskulatur ausgebildet ist. Derartige Längeneinstellungseinrichtungen sind in vielfältiger Weise bekannt, weshalb sie hier nicht näher dargestellt und beschrieben sind. Selbstverständlich sind auch andere bekannte Längeneinstellungseinrichtungen denkbar, die für einen derartigen Einsatz geeignet sind.

Bei einer Verletzung des vorderen Kreuzbandes können die obere Halbschale 6 und untere Halbschale 9 der erfindungsgemässen Knieorthese 1 so in die Knieorthese 1 eingesetzt werden, dass die obere Halbschale 6 von hinten auf den Oberschenkel aufgesetzt werden kann, während die untere Halbschale 9 von vorn auf den Unterschenkel aufgesetzt werden kann. Die Umlenkung des längsstabilen flexiblen Zugelementes 17 erfolgt dann in die ventrale Richtung. Beim Beugen des Knies wird somit die untere Halbschale 6 um die Schwenkachse 15 dorsal gegen das Kniegelenk gedrückt, die Tibia wird somit bezüglich dem Femur ebenfalls in dorsaler Richtung gedrückt, was zu einer Entlastung des vorderen Kreuzbandes führt.

Mit dieser erfindungsgemässen Knieorthese kann eine optimale Abstützung des Kniegelenks erreicht werden, insbesondere wenn eine Verletzung des hinteren oder vorderen Kreuzbandes vorliegt, durch diese Knieorthese wird das Kniegelenk insbesondere dann in optimaler Weise abgestützt, wenn es gebeugt wird.

## Patentansprüche

1. Knieorthese zur Stützung eines Kniegelenks bei einer Instabilität, umfassend einen am Oberschenkel über Befestigungsmittel (7) befestigbaren Oberschenkelteil (4) mit einer oberen Halbschale (6) und an der oberen Halbschale (6) befestigte Gelenkplatten (14), einen am Unterschenkel über Befestigungsmittel (10) befestigbaren Unterschenkelteil (5) mit einer unteren Halbschale (9) und seitlich an der unteren Halbschale (9) angebrachten Schienenelemente (12), welche Schienenelemente (12) über Gelenkmittel (13) mit der jeweiligen Gelenkplatte (14) verbunden sind und in welchen Schienenelementen (12) an den den Gelenkmitteln (13) abgewandten Bereichen die untere Halbschale (9) um eine Schwenkachse (15) schwenkbar gelagert ist und über Stellmittel (16) gegen den Unterschenkel hin schwenkbar ist, die Stellmittel (16) aus mindestens einem längsstabilen flexiblen Zugelement (17) gebildet sind, dessen einer Endbereich an einem bezüglich der Schienenelemente (12) schwenkbaren Teil der Gelenkmittel (13) mittels einer ersten Halterung (18) gehalten ist, dessen anderer Endbereich an der unteren Halbschale (9) mittels einer zweiten Halterung (19) gehalten ist und welches längsstabile flexible Zugelement (17) zwischen der ersten Halterung (18) und der zweiten Halterung (19) um ein am Schienenelement (12) angebrachtes Umlenkelement (20) umgelenkt ist.

2. Knieorthese zur Stützung eines Kniegelenks nach Anspruch 1, **dadurch gekennzeichnet, dass** die Gelenkmittel (13) jeweils als Viergelenksystem (27) ausgebildet sind mit einem ersten Schenkel (22) und einem zweiten Schenkel (24), welche jeweils an der Gelenkplatte (14) und am entsprechenden Schienenelement (12) angelenkt sind.

3. Knieorthese zur Stützung eines Kniegelenks nach Anspruch 2, **dadurch gekennzeichnet, dass** die erste Halterung (18) zum Halten des längsstabilen flexiblen Zugelements (17) am ersten Schenkel (22) des Viergelenksystems (27) angebracht ist.

4. Knieorthese zur Stützung eines Kniegelenks nach Anspruch 3, **dadurch gekennzeichnet, dass** die erste Halterung (18) zum Halten des längsstabilen flexiblen Zugelements (17) verstellbar am ersten Schenkel (22) des Viergelenksystems (27) angebracht ist.

5. Knieorthese zur Stützung eines Kniegelenks nach Anspruch 3 oder 4, **dadurch gekennzeichnet, dass** zur Verstellung der ersten Halterung (18) am ersten Schenkel (22) eine schlitzförmige Ausnehmung (28) angebracht ist, entlang welcher die erste Halterung (18) verschiebbar und in der eingestellten Position fixierbar ist.

6. Knieorthese zur Stützung eines Kniegelenks nach Anspruch 5, **dadurch gekennzeichnet, dass** die erste Halterung (18) in einem Gleitschuh (29) angebracht ist, welcher entlang der schlitzförmigen Ausnehmung (28) verschiebbar ist und durch Klemmmittel (30, 31) fixierbar ist.

7. Knieorthese zur Stützung eines Kniegelenks nach Anspruch 5 oder 6, **dadurch gekennzeichnet, dass** entlang der schlitzförmigen Ausnehmung (28) eine Skala (32) angeordnet ist.

8. Knieorthese zur Stützung eines Kniegelenks nach einem der Ansprüche 1 bis 7, **dadurch gekennzeichnet, dass** in das längsstabile flexible Zugelement (17) mindestens ein Federelement (35) eingesetzt ist.

9. Knieorthese zur Stützung eines Kniegelenks nach einem der Ansprüche 1 bis 8, **dadurch gekennzeichnet, dass** die Länge des längsstabilen flexiblen Elementes (17) über eine Längeneinstellungseinrichtung (36) einstellbar ist.

10. Knieorthese zur Stützung eines Kniegelenks nach Anspruch 9, **dadurch gekennzeichnet, dass** die Längeneinstellungseinrichtung (36) in die zweite Halterung (19) integriert ist und als drehbarer Knopf (37) ausgebildet ist, dessen Achse (38) in der unteren Halbschale (9) gelagert ist und um welche das längsstabile flexible Zugelement (17) wickelbar ist.

11. Knieorthese zur Stützung eines Kniegelenks nach einem der Ansprüche 1 bis 10, **dadurch gekennzeichnet, dass** die Befestigungsmittel (7, 10) für die obere Halbschale (6) und untere Halbschale (9) als Bänder (8, 11) mit Klettverschluss ausgebildet sind.

12. Knieorthese zur Stützung eines Kniegelenks nach einem der Ansprüche 1 bis 11, **dadurch gekennzeichnet, dass** das längsstabile flexible Zugelement (17) ein Kabel ist.

## Claims

1. Knee orthosis for support of a knee joint in the case of instability, comprising a thigh portion (4), attachable on the thigh via fixing means (7), with an upper half shell (6) and hinge plates (14) attached to the upper half shell (6), a lower leg portion (5) attachable on the lower leg via fixing means (10) with a lower half shell (9) and splint elements (12) attached laterally on the lower half shell (9), which splint elements (12) are connected via articulation means (13) to the respective hinge plate (14) and in which splint elements (12), in the regions remote from the articulation means (13), the lower half shell (9) is pivotably borne about a pivot axis (15) and is pivotable, via adjustment means (16), toward the lower leg, where the adjustment means (16) are formed by at least one longitudinally stable, flexible traction element (17), whose one end region is held on a member of the articulation means (13) pivotable with respect to the splint elements (12) by means of a first holding device (18), whose other endregion is held on the lower half shell (9) by means of a second holding device (19) and which longitudinally stable, flexible traction element (17) is diverted between the first holding device (18) and the second holding device (19) about a diversion element (20) mounted on the splint element (12).

2. Knee orthosis for support of a knee joints according to claim 1, **characterized in that** the articulation means (13) are designed in each case as four joint system (27) with a first member (22) and a second member (24), which are each linked to the hinge plate (14) and to the corresponding splint element (12).

3. Knee orthosis for support of a knee joint according to claim 2, **characterized in that** the first holding device (18) is mounted for holding the longitudinally stable, flexible traction element (17) on the first member (22) of the four joint system (27).

4. Knee orthosis for support of a knee joint according to claim 3, **characterized in that** the first holding device (18) for holding the longitudinally stable, flexible traction element (17) is adjustably mounted on the first member (22) of the four joint system (27).

5. Knee orthosis for support of a knee joint according to claim 3 or 4, **characterized in that** for adjusting the first holding device (18) on the first member (22) a slot-shaped recess (28) is provided along which the first holding device (18) is slidable and fixable in the set position.

6. Knee orthosis for support of a knee joint according to claim 5, **characterized in that** the first holding device (18) is mounted in a sliding block (29) which is slidable along the slot-shaped recess (28) and is fixable through clamping means (30, 31).

7. Knee orthosis for support of a knee joint according to claim 5 or 6, **characterized in that** a scale (32) is disposed along the slot-shaped recess (28).

8. Knee orthosis for support of a knee joint according to one of the claims 1 to 7, **characterized in that** at least one spring element (35) is inserted in the longitudinally stable, flexible traction element (17).

9. Knee orthosis for support of a knee joint according to one of the claims 1 to 8, **characterized in that** the length of the longitudinally stable, flexible tracting element (17) is adjustable via a length adjustment device (36).

10. Knee orthosis for support of a knee joint according to claim 9, **characterized in that** the length adjustment device (36) is integrated in the second holding device (19) and is designed as rotatable knob (37) whose shaft (38) is borne in the lower half shell (9) and about which the longitudinally stable, flexible tracting element (17) is able to be wound.

11. Knee orthosis for support of a knee joint according to one of the claims 1 to 10, **characterized in that** the fixing means (7, 10) for the upper half shell (6) and lower half shell (9) are designed as bands (8, 11) with hook-and-loop fastener.

12. Knee orthosis for support of a knee joint according to one of the claims 1 to 11, **characterized in that** the longitudinally stable, flexible element (17) is a cable.

## Revendications

1. Orthèse de genou destinée au soutien d'une articulation du genou en cas d'instabilité, comprenant une partie fémorale (4), pouvant être fixée à la cuisse à l'aide de moyens de fixation (7), pourvue d'une moitié de coque supérieure (6) et de plaques articulées (14) attachées à la moitié de coque supérieure (6), une partie de jambe inférieure (5) pouvant être fixée à la jambe inférieure par des moyens de fixation (10), pourvue d'une moitié de coque inférieure (9) et d'éléments d'attelle (12), lesquels éléments d'attelle (12) étant reliés aux plaques articulées (14) par le biais de moyens d'articulation (13), et dans lesquels éléments d'attelle (12), dans les zones distantes des moyens d'articulation (13), la moitié de coque inférieure (9) est logée de manière pivotante autour d'un axe de pivot (15), et peut être pivotée, grâce aux moyens d'ajustement (16), en direction de la jambe inférieure, où les moyens d'ajustement (16) sont formés par au moins un élément de traction flexible (17) stable longitudinalement, dont une première partie d'extrémité est maintenue sur une pièce (22) des moyens d'articulation (13) qui peut être pivotée par rapport aux éléments d'attelle (12) grâce à un premier dispositif de maintien (18), et dont une autre partie est maintenue sur la moitié de coque inférieure (9) au moyen d'un deuxième dispositif de maintien (19), lequel élément de traction flexible (17) stable longitudinalement étant dévié entre le premier dispositif de maintien(18) et le deuxième dispositif de maintien(19) par un élément de déviation (20) monté sur l'élément d'attelle (12).

2. Orthèse de genou destinée au soutien d'une articulation du genou selon la revendication 1, **caractérisée en ce que** les moyens d'articulation (13) sont conçus respectivement comme un système de joint quadruplement articulé (27), avec une première branche (22) et une deuxième branche (24), qui sont reliées respectivement à la plaque articulée (14) et à l'élément d'attelle (12) correspondant.

3. Orthèse de genou destinée au soutien d'une articulation du genou selon la revendication 2, **caractérisée en ce que** le premier dispositif de maintien (18) est monté pour tenir l'élément de traction flexible stable longitudinalement (17) sur la première branche (22) du système de joint quadruplement articulé (27).

4. Orthèse de genou destinée au soutien d'une articulation du genou selon la revendication 3, **caractérisée en ce que** le premier dispositif de maintien (18) pour maintenir l'élément de traction flexible stable longitudinalement (17) est monté de manière ajustable sur la première branche (22) du système de joint quadruplement articulé (27).

5. Orthèse de genou destinée au soutien d'une articulation du genou selon la revendication 3 ou 4, **caractérisée en ce qu'**un évidement en forme de fente (28) est aménagée sur la première branche (22) pour permettre l'ajustement du premier dispositif de maintien (18), et le long de laquelle le premier dispositif de maintien (18) peut coulisser et être fixée dans la position réglée.

6. Orthèse de genou destinée au soutien d'une articulation du genou selon la revendication 5, **caractérisée en ce que** le premier dispositif de maintien (18) est monté dans un patin de guidage (29), qui peut coulisser le long de l'évidement en forme de fente (28), et est fixable par l'intermédiaire de moyens de serrage (30, 31).

7. Orthèse de genou destinée au soutien d'une articulation du genou selon la revendication 5 ou 6, **caractérisée en ce qu'**une échelle (32) est disposée le long de l'évidement en forme de fente (28).

8. Orthèse de genou destinée au soutien d'une articulation du genou selon l'une des revendications 1 à 7, **caractérisée en ce qu'**au moins un élément amortisseur (35) est inséré dans l'élément longitudinal flexible stable longitudinalement (17).

9. Orthèse de genou destinée au soutien d'une articulation du genou selon l'une des revendications 1 à 8, **caractérisée en ce que** la longueur de l'élément flexible stable longitudinalement (17) est ajustable grâce à un dispositif d'ajustement de longueur (36).

10. Orthèse de genou destinée au soutien d'une articulation du genou selon la revendication 9, **caractérisée en ce que** le dispositif d'ajustement de longueur (36) est intégré dans le deuxième dispositif de maintien (19) et est conçu comme un bouton tournant (37), dont l'axe (38) est logé dans la moitié de coque inférieure (9), et autour duquel l'élément flexible stable longitudinalement (17) peut être enroulé.

11. Orthèse de genou destinée au soutien d'une articulation du genou selon l'une des revendications 1 à 10, **caractérisée en ce que** les moyens de fixation (7, 10) pour la moitié de coque supérieure (6) et la moitié de coque inférieure (9) sont conçus comme des bandes (8, 11) avec fermeture autoagrippante de type velcro.

12. Orthèse de genou destinée au soutien d'une articulation du genou selon l'une des revendications 1 à 11, **caractérisée en ce que** l'élément flexible stable longitudinalement (17) est un câble.
